# EUROPEAN PATENT SPECIFICATION

(11) **EP 4 566 656 B1**
(45) Date of publication and mention of the grant of the patent: **13.05.2026**
(21) Application number: 24217352.4
(22) Date of filing: 04.12.2024
(51) Int. Cl.: A61N 1/368, A61B 5/349

(54) **SYSTEMS FOR MANAGING ATRIAL-VENTRICULAR DELAY**
SYSTEME ZUR VERWALTUNG VON VORHOFVENTRIKULÄRER VERZÖGERUNG
SYSTÈMES DE GESTION DE RETARD AURICULO-VENTRICULAIRE

(30) Priority: 06.12.2023 US 202363606981 P; 02.12.2024 US 202418965595
(43) Date of publication of application: 11.06.2025
(73) Proprietor: Pacesetter, Inc., Sylmar, CA 91342 (US)
(72) Inventor: Mangual-Soto, Jan O, Sylmar, CA 91342 (US); Simonetti, Angelo, Sylmar, CA 91342 (US)
(74) Representative: Barker Brettell Sweden AB

(56) References cited:
- US-B2- 11 273 312
- US-B2- 8 509 891
- US-B2- 9 220 905

## Description

### TECHNICAL FIELD

This disclosure relates generally to implantable cardiac stimulating devices.

### BACKGROUND

In a normal human heart, the sinus node, generally located near the junction of the superior vena cava and the right atrium, constitutes the primary natural pacemaker initiating rhythmic electrical excitation of the heart chambers. The cardiac impulse, or excitation pulse, arising from the sinus node is transmitted to the two atrial chambers, causing a depolarization known as a P-wave and atrial chamber contractions. The excitation pulse is further transmitted to and through the ventricles via the atrioventricular (AV) node and a ventricular conduction system, causing a depolarization and ventricular chamber contractions. The ventricular conduction system includes the bundle of HIS (also referred to as the HIS bundle), the left and right bundle branches, and the Purkinje fibers. The depolarization of the interventricular septum and ventricles is generally referred to as a QRS complex. The QRS complex is observed and measured through the use of electrocardiogram (ECG) machines and similar equipment for measuring electrical activity of the heart.

Disruption of this natural pacemaking and conduction system as a result of aging or disease can be successfully treated by artificial cardiac pacing using implantable cardiac stimulation devices, including pacemakers and implantable defibrillators. The implantable cardiac stimulation devices deliver rhythmic electrical pulses or other anti-arrhythmia therapies to the heart at a desired energy and rate via electrodes implanted in contact with the heart tissue. To the extent the electrical pulses are sufficient to induce depolarization of the associated heart tissue, the heart tissue is said to be captured. The minimum electrical pulse resulting in capture is generally referred to as the capture threshold.

In the majority of individuals, the most effective heartbeat is triggered by the patient's own natural pacing physiology. Implantable cardiac stimulation devices are intended as a back-up when the natural pacing functionality of the patient's heart fails or acts inefficiently (such as in cases of sinus arrest and symptomatic bradycardia, respectively) or when the heart's conduction system fails or acts inefficiently. In a large number of heart failure (HF) patients, natural conduction through the AV node and the HIS bundle are intact and disruption of ventricular rhythm is the result of conduction disorders residing in the left and/or right bundle branches.

Dilation of the heart due to congestive heart failure (CHF) has been associated with delayed conduction through the ventricles. This delayed conduction leads to reduced hemodynamic efficiency of the failing heart because of the resulting poor synchronization of the heart chambers.

Direct stimulation of the HIS bundle has been found to provide hemodynamic improvement for various patients including those suffering from dilated cardiomyopathy but having normal ventricular activation. However, a significant learning curve remains in connection with the implant procedure and in some instances involves a higher rate of lead placement adjustment. HIS bundle pacing (HBP) is also associated with higher capture thresholds and lower ventricular sensing amplitudes as compared to other pacing modes, which can be challenging for device functionality and battery longevity.

Further, HBP may exhibit lower success in patients with His-Purkinje conduction disease when the conduction block is more distal in the conduction system. Transvenous left bundle branch (LBB) pacing has been proposed where the lead is implanted from the right ventricle and screwed deep into the septum to deliver pacing stimuli at the LBB. The LBB area pacing technique has been shown to be associated with an easier implant procedure, lower capture thresholds, and higher ventricular sensing amplitudes compared to HBP. LBB area pacing has been proposed to be used in parallel or in lieu of HBP to increase overall success of physiological pacing.

In addition, the narrowing of QRS duration (QRSd) has been proven to predict long-term mortality reduction in patients utilizing LBB pacing. A dynamic atrioventricular delay (AVD) optimization algorithm based on the promoting of biventricular fusion pacing has been proven to narrow QRSd over biventricular pacing with fixed AVD. In HF patients treated with LBB area pacing, QRSd narrowing may be a predictor of patient outcome and a target. As a result, a need exists for systems and methods that utilize a dynamic algorithm for AVD optimization that narrows QRSd in patients treated with LBB area pacing.

US9220905 discloses an implantable medical device for managing therapy.

### SUMMARY

The present invention is defined in the independent claim. Further embodiments are defined in dependent claims.

In accordance with embodiments herein, an implantable medical device (IMD) for managing therapy is provided that comprises a connector coupleable to a lead with an electrode, a memory configured to store program instructions and one or more processors. The one or more processors can be configured to execute the program instructions to determine a sensed right atrium (RAs) event or a paced right atrial (RAp) event (RAs,p event), determine a sensed right ventricle (RVs) event by detecting a cardiac activity (CA) signal reaches a threshold amplitude and determining a maximum amplitude in a determined period of time after the CA signal reaches the threshold amplitude, and determine an RAs,p-RVs interval between the RAs,p event and RVs event. The one or more processors can also be configured to calculate an atrioventricular delay (AV) delay based on the RAs,p-RVs interval, and manage therapy, provided by the IMD, based on the AV delay that is calculated.

In one embodiment, the one or more processors can be configured to manage the therapy by controlling the electrode to deliver left bundle branch area pacing therapy based on the AV delay.

In one embodiment, the IMD can be a dual chamber implantable pulse generator (IPG).

In one embodiment, the one or more processors may be configured to calculate the AV delay by subtracting a determined percentage of the RAs,p-RVs interval from the RAs,p-RVs interval.

In one embodiment, the CA signal can be an intrinsic CA signal.

In one embodiment, the one or more processors can be further configured to after calculating the AV delay, set the AV delay as an initial AV delay and utilize the initial AV delay to manage the therapy for a determined number of CA signals.

In one embodiment, the one or more processors can be further configured to count a number of the CA signals after a first CA signal and compare a number of counted CA signals to the determined number of CA signals.

In one embodiment, the one or more processor can also be configured to repeat determination of the RAs,p event, determination of the RVs event, determination of the RAs,p-RVs interval and calculation of the AV delay after the number of counted CA signals matches the determined number of CA signals, to calculate an updated AV delay and manage the therapy provided by the IMD, based on the updated AV delay.

In one embodiment, the one or more processors are further configured to reset a count of the number of counted CA signals in response to calculating the updated AV delay.

In one embodiment, the one or more processors can be further configured to after calculating the AV delay, determine a second RAs,p-RVs interval based on a second CA signal, compare the second RAs,p-RVs interval to the AV delay, and responsive to the second RAs,p-RVs interval being less than the AV delay, calculate an updated AV delay based on the second RAs,p-RVs interval.

In one embodiment, the one or more processors can be further configured to count a number of CA signals obtained after a first CA signal, compare a counted number of CA signals to a determined number of CA signals, and reset the counted number of the CA signals in response to calculating the updated AV delay.

In one embodiment, the one or more processors can be further configured to manage the therapy, provided by the IMD, based on the updated AV delay, and the one or more processors can also be configured to manage the therapy by controlling the electrode to deliver left bundle branch area pacing therapy based on the updated AV delay.

In one embodiment, the IMD further comprises a sensing circuitry configured to be coupled to the electrode to detect the CA signal.

In one embodiment, the sensing circuitry is configured to sense the RAs event.

In one embodiment, the IMD further comprises a pulse generator controlled by the one or more processors to deliver pacing stimulation pulses via the electrode.

In one embodiment, the pulse generator is configured to pace the right atrium.

In one embodiment, the one or more processors are further configured to control the pulse generator to deliver a pacing stimulation pulse via the electrode at the expiry of the AV delay.

In one embodiment, the one or more processors are configured to determine the RVs event as the maximum amplitude in the CA signal in the determined period of time after the CA signal reaches the threshold amplitude.

### BRIEF DESCRIPTION OF THE DRAWINGS

The various features of the present disclosure and the manner of attaining them will be described in greater detail with reference to the following description, claims, and drawings, wherein reference numerals are reused, where appropriate, to indicate a correspondence between the referenced items, and wherein:
FIG. 1 is schematic diagram of myocardial depolarization resulting from an intrinsic heartbeat, and different pacing conditions.
FIG. 2 is simplified, partly cutaway view illustrating an IMD implanted into a patient's heart for delivering therapy.
FIG. 3 illustrates a simplified block diagram of the IMD, which is capable of treating arrhythmias with stimulation therapy.
FIG. 4 illustrates a graph of a CA signal in accordance with embodiments herein.
FIG. 5 illustrates a graph of a CA signal in accordance with embodiments herein.
FIG. 6 illustrates a graph of a CA signal in accordance with embodiments herein.
FIG. 7 illustrates a process for managing stimulation timing for the electrode configuration shown in FIG. 2.

### DETAILED DESCRIPTION

It will be readily understood that the components of the embodiments as generally described and illustrated in the Figures herein may be arranged and designed in a wide variety of different configurations in addition to the described example embodiments. Thus, the following more detailed description of the example embodiments, as represented in the Figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The non-claimed methods described herein may employ structures or aspects of various embodiments (e.g., devices, systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

The terms "cardiac activity signal", "cardiac activity signals", "CA signal" and "CA signals" (collectively "CA signals") are used interchangeably herein to refer to measured signals indicative of cardiac activity by a region or chamber of interest in the heart. The cardiac activity may be normal/healthy or abnormal/arrhythmic. An example of CA signals includes EGM signals. Electrical based CA signals refer to an analog or digital electrical signal recorded by two or more electrodes, where the electrical signals are indicative of cardiac activity.

The phrase "conduction system of a heart", "conduction system of the heart", or "conduction system" are used interchangeably throughout to refer to any portion, segment, part, etc. of the heart related to conduction of the heart. Conduction systems can include the right bundle branch (RBB), LBB, HIS, right atrium, right ventricle, or the like. To this end, when an electrode is described as positioned proximate to a conduction system of the heart, the electrode is considered to be positioned at, adjacent, on, next to, one of the RBB, LBB, HIS, right atrium, right ventricle, or the like.

The term "RAs,p-RVs interval", as used herein, refers to a measured intrinsic conduction time from either a sensed right atrial (RAs) event or a paced right atrial (RAp) event (e.g., RAs,p event) to a sensed right ventricular (RVs) event. The alternative RAs and RAp events are collectively referred to as an RAs,p event. The RAs event may occur at the time that a CA signal first reaches (e.g., crosses) a threshold voltage amplitude. The threshold voltage amplitude (e.g., sensing threshold) may indicate that the CA signal has been sensed. The RAp event may occur at the time that a pacing pulse is delivered by an electrode of an implantable lead to the right atrium. In an example, the RVs event occurs during a predetermined period of time after the CA signal reaches the RV. More specifically, the predetermined period of time may start when the CA signal reaches a threshold voltage amplitude, such as the same threshold voltage amplitude as the RAs, or another threshold voltage amplitude associated with the RV. In one example, the RVs event may occur at a time corresponding to a maximum voltage amplitude of the CA signal during a determined period of time. The determined period of time can be 100 ms, 120 ms, 150 ms, 180 ms, 200 ms, etc.

The term "RAs,p event", as used herein, refers to an RAs event or an RAp event. For the avoidance of doubt, the RAs,p event is a single event that results from either a naturally produced CA signal or a paced produced CA signal.

The terms "atrioventricular delay", "AV delay" and "AVD" can be used interchangeably herein, and each refer to a programmed time delay to be used by the implantable medical device in connection with delivering therapy. In one example the AVD can be calculated by applying a hysteresis offset to the RAs,p-RVs interval that is determined. The AVD may be a delay after a detected atrial signal or paced event until the delivery of a stimulation pulse, such as a pacing pulse to the LBB area.

The term "event" as used herein refers to a portion or part of a CA signal resulting from electrical properties of the heart. For example, maximum amplitudes, local maximum amplitudes, minimum amplitudes, local minimum amplitudes, or the like are each an event. In an example P-waves, R-waves, and depolarization signals associated with fibrillation are each an event.

The term "characteristics of interest" and "COls" when used herein related to CA signals shall refer to characteristics, parameters, and/or features of the CA signals that can be measured. Characteristics of interest can include measurements at a point, CA signal morphology, parameters that can be calculated, or the like. In example embodiments, COIs may include amplitude, QRS intervals, RAs event times, RVs event times, RAs,p-RVs intervals, AVDs, or the like. In addition, when used in association with a location, such as an RA or RV, including when provided as a RA COI and a RV COI means the COI relates to a measurement, parameter, feature, etc. of that location. An example RA COI can be where a CA signal reaches a threshold value associated with an intrinsic or artificial pulse reaching the RA location.

The term "intrinsic AVD" as used herein refers to an intrinsic time delay that is naturally provided by the functioning of the heart.

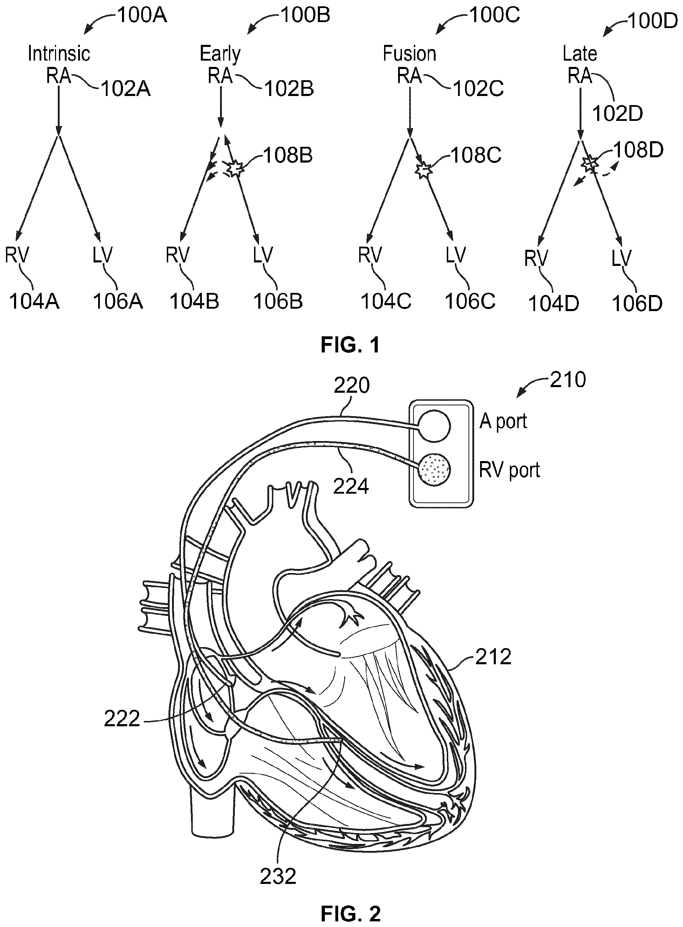

The term "fusion pacing" as used herein refers to pacing in the LBB area simultaneously with intrinsic activation along the right bundle branch (RBB). In example embodiments, the fusion results in QRS narrowing. In one example, FIG. 1 illustrates a schematic representation of myocardial depolarization 1) when no pacing is provided and only an intrinsic RA, RV and LV activation 100A is provided, 2) when an early LBB area pacing activation 100B is provided, 3) when a fusion pacing activation 100C occurs, and 4) when a late LBB area pacing activation 100D occurs. As illustrated, each activation is for providing depolarization from the RA 102A-102D to a RV 104A-D and LV 106A-D, and when pacing occurs the LBB is stimulated. Early pacing 108B (as illustrated as 100B) stimulates the LBB directly and the RBB indirectly, resulting in suboptimal QRS narrowing. Meanwhile, late pacing 108D (as illustrated a 100D) finds the RBB and LBB in a refractory period, so the pacing 108D does not contribute to depolarization. In contrast, fusion pacing 108C (as illustrated as 100C) provides pacing in the LBB area concurrently (e.g. contemporaneously) with intrinsic activation along the RBB, resulting in enhanced QRS narrowing.

Embodiments may be implemented in connection with one or more implantable medical devices (IMDs). Non-limiting examples of IMDs include one or more of implantable leadless monitoring and/or therapy devices, and/or alternative implantable medical devices. For example, the IMD may represent a cardiac monitoring device, pacemaker, cardioverter, cardiac rhythm management device, defibrillator, neurostimulator, leadless monitoring device, leadless pacemaker and the like. For example, the IMD may include one or more structural and/or functional aspects of the device(s) described in U.S. Patent 9,333,351 "Neurostimulation Method And System To Treat Apnea" and U.S. Patent 9,044,610 "System And Methods For Providing A Distributed Virtual Stimulation Cathode For Use With An Implantable Neurostimulation System." Additionally or alternatively, the IMD may be a leadless implantable medical device (LIMD) that include one or more structural and/or functional aspects of the device(s) described in U.S. Patent 9,216,285 "Leadless Implantable Medical Device Having Removable And Fixed Components" and U.S. Patent 8,831,747 "Leadless Neurostimulation Device And Method Including The Same". Additionally or alternatively, the IMD may include one or more structural and/or functional aspects of the device(s) described in U.S. Patent 8,391,980 "Method And System For Identifying A Potential Lead Failure In An Implantable Medical Device" and U.S. Patent 9,232,485 "System And Method For Selectively Communicating With An Implantable Medical Device".

The present disclosure is directed to various aspects of stimulation devices and corresponding non-claimed methods related to managing AV delay during pacing. Aspects of the present disclosure may be implemented in a dual chamber, cardiac resynchronization therapy and/or other implantable devices. Embodiments herein may be implemented in connection with the methods, systems and devices described in U.S. Patent No. 11,154,719, titled "METHOD AND SYSTEM UTILIZING A PERCENTAGE-BASED ATRIO-VENTRICULAR DELAY ADJUSTMENT", filed 18-September 18-2019 and issued 26-October-2021, United States Patent No. 11,273,312, titled "METHOD AND SYSTEM FOR DYNAMIC DEVICE-BASED ADJUSTMENT", filed 18-September-2019 and issued 15-March-2022, United States Patent No. 11,654,288, titled "SYSTEMS AND METHOD FOR MANAGING ATRIAL-VENTRICULAR DELAY ADJUSTMENTS", filed 10-August-2020 and issued 23-May-2023, United States Patent No. 11,745,018, titled "METHOD AND SYSTEM FOR DYNAMIC DEVICE-BASED DELAY ADJUSTMENT", filed 1-February-2022 and issued 5-September-2023.

A non-claimed method is proposed for dynamic adjustments of the AVD in particular for an IMD in the form of a dual chamber implantable pulse generator (IPG) during left bundle block area pacing (LBBAP). In particular, when fixed AVD parameters are programmed, the fixed parameters may not be ideal for out-of-clinic maintenance of the QRS duration. A non-claimed method is proposed that allows updating the AVD to promote pacing in the LBB area and fusion with intrinsic activation down the right bundle branch (RBB). The proposed method may be based in part on intrinsic AVD. The proposed method aims to promote, in a dynamic out-of-clinic manner, fusion pacing during LBBAP for optimal QRS narrowing. By comparison, early pacing stimulates the LBB directly and the RBB indirectly, resulting in suboptimal QRS narrowing. Late pacing finds the RBB and LBB in refractory period, so does not contribute to depolarization. Beneficially, fusion pacing occurs when pacing the LBB area concurrently with intrinsic activation along the right bundle branch (RBB), resulting in QRS narrowing.

Dynamic monitoring of the sensed RA-RV interval (e.g. RAs,p-RVs) can be used to time the pacing in the LBB area to promote fusion pacing. To measure the RAs,p-RVs interval, the dynamic pacing management algorithm described herein does not time the RVs event at the first crossing of a detection threshold by the CA signal, but rather uses the maximum or peak value of the CA signal within a determined RV period of time. The determined RV period of time is a designated time period extending from the CA signal crossing a detection threshold.

By using the maximum value the invention can be used with pacemaker devices that have both bipolar and unipolar sense configuration with automatic or fixed sensitivity. When using the current method for measuring RAs,p-RVs intervals, there might be a difference in terms of a few milliseconds (ms) switching from bipolar to unipolar sense configurations and vice versa because in unipolar configuration the signal may be wider than the bipolar configuration and the RV sense time may be earlier, particularly if sensibility is programmed as fixed and there is a substantial difference in R-wave sensing. Measuring the RAs,p-RVs interval to the peak of the R-wave signal gives a complete view of ventricular activation and makes the pacing management algorithm more accurate.

Initially, when activated, the pacing management algorithm described herein can measure an intrinsic RAs,p-RVs sense interval using the peak of the R-wave signal as the RVs CA signal. If no intrinsic activation is detected, then pacing may be delivered to the LBBAP lead with a fixed AVD such as 110 ms, 140 ms, etc.

If intrinsic activation is detected, the system can deliver LBBAP at a programmable offset of the RAs,p-RVs interval. The programmable offset may be a percentage of the RA-RV sense interval, such as 5%, 10%, 15%, 20%, 25%, in a range between 5%-25%, or the like. In an example, the AVD may be calculated as the RAs,p-RVs interval minus the hysteresis offset. In this example when the offset is 10%, the calculated AVD may be the value of the RAs,p-RVs interval minus 0.1*the value of the RAs,p-RVs interval, so AVD=X-0.1X. Generally, the AVD may be calculated as X-αX, wherein X denotes the RAs,p-RVs interval and α is a non-zero value smaller than one, i.e., 0<α<1. In a preferred embodiment, α is selected within an interval of from 0.05 up to 0.25, preferably selected within an interval of from 0.1 up to 0.15. The offset promotes fusion pacing, meaning pacing the LBBAP lead, and intrinsic activation down the RBB to achieve a narrower QRS. The pacing management algorithm preferably keeps the programmed AVD up to N beat cycles (BC) (e.g., BC = N = 255). In examples N = between 225-275, and preferably between 245-265. If the pacing management algorithm uses a detected RV intrinsic activation before N beat cycles, a new searching and recording of the RAs,p-RVs interval may be started and a new AVD may be calculated and programmed. Instead, if the pacing management algorithm does not detect any intrinsic activations, the RAs,p-RVs interval may not be updated until after the Nth beat cycle.

### Overview of System and Components

Figure 2 shows an exemplary IMD 210 that is implanted into the patient as part of the implantable cardiac system. The IMD 210 in one example embodiment is a dual chamber implantable pulse generator (IPG). The IMD 210 may be implemented as a full-function dual-chamber pacemaker, equipped with both atrial and ventricular sensing, pacing circuitry, and treatment circuitry for four chamber sensing and stimulation therapy (including both pacing and shock treatment). Alternatively, the IMD 210 may be implemented with a reduced set of functions and components. For instance, the IMD may be implemented without ventricular pacing.

The IMD 210 can be in electrical communication with a patient's heart 212 by way of two leads, 220 and 224, and suitable for delivering multi-chamber stimulation and pacing and depolarization therapy. In example embodiments, the leads 220, 224 may be placed proximate to any conduction system of the heart to provide the sensing, pacing, therapy, etc. as described herein. In this example embodiment, to sense ventricular CA signals and to provide LBB area stimulation therapy, the pacing device 210 is coupled to an implantable lead 224 having at least a ventricular tip electrode 232. The tip electrode 232 may be implanted in the patient's LBB of the HIS bundle.

Accordingly, the lead 224 is capable of receiving depolarization signals propagated in the LBB and exiting the Purkinje fibers to the myocardium and/or delivering stimulation to the LBB area, creating a depolarization that can be propagated through the lower conductive pathways of the right and left ventricles (i.e., the right and left bundle branches and Purkinje fibers). To this end, an exemplary implantable lead 224 is designed to receive ventricular cardiac signals and to deliver LBB area pacing therapy using at least the ventricular tip electrode 232.

In an example application, the lead 224 may be transvenously inserted into the heart 212 so that a portion of the lead 224 is located within the right ventricle. When the tip electrode 232 is in the LBB area, a coil electrode of the lead 224 may be positioned in the right ventricle and a superior vena cava (SVC) coil electrode of the lead 224 may be positioned in the SVC of the heart 212. The lead 220 may be an atrial lead that is also located in the SVC. The atrial lead 220 has a tip electrode 222. In this manner, atrial CA signals can be received by the IMD 210.

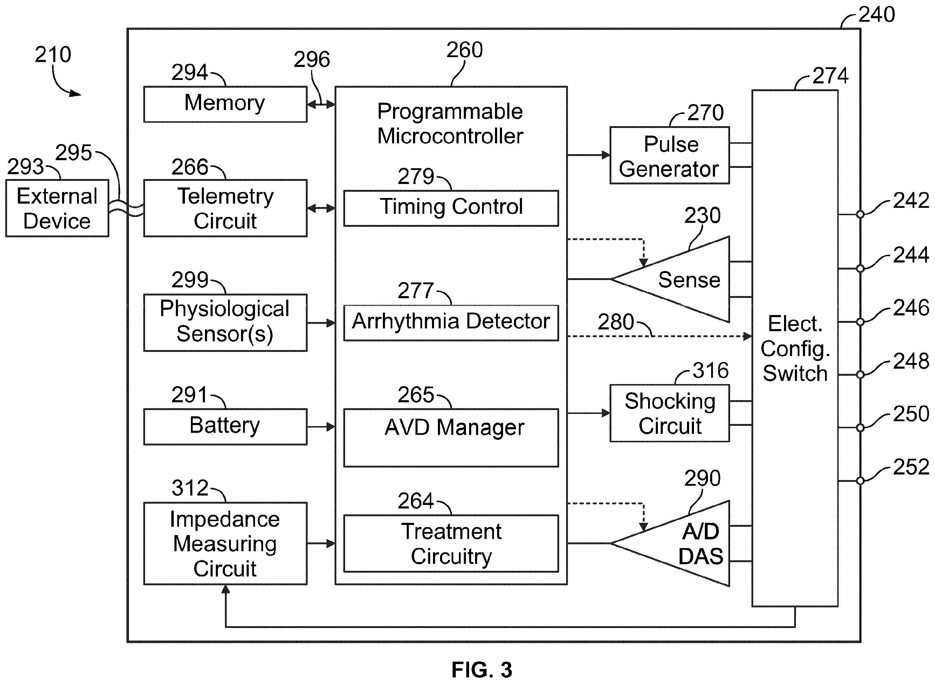

Figure 3 illustrates a simplified block diagram of the multi-chamber IMD 210, which is capable of treating both fast and slow arrhythmias with stimulation therapy, including cardioversion, defibrillation, and/or pacing stimulation. While a particular multi-chamber device is shown, this is for illustration purposes only, and one of skill in the art could readily duplicate, eliminate or disable the appropriate circuitry in any desired combination to provide a device capable of treating the appropriate chamber(s) with cardioversion, defibrillation and pacing stimulation.

The housing 240 for the IMD 210 is often referred to as the "can", "case" or "case electrode" and may be programmably selected to act as the return electrode for all "unipolar" modes. The housing 240 may further be used as a return electrode alone or in combination with one or more coil electrodes for shocking purposes. The housing 240 further includes a connector (not shown) having a plurality of terminals 242, 244, 246, 248, 250, and 252 (shown schematically). To achieve right atrial sensing and LBB area pacing, the connector includes at least a right atrial tip terminal (AR TIP) 242 adapted for connection to the atrial tip electrode 222 (shown in Figure 2). To achieve ventricular chamber sensing, the connector includes at least a right ventricular tip terminal (VR TIP) 252 adapted for connection to the tip electrode 232 of the lead 224.

At the core of the IMD 210 is a programmable microcontroller 260 which controls the various modes of stimulation and pacing therapy. The microcontroller 260, also referred to as processor herein, includes a microprocessor, or equivalent control circuitry, designed specifically for controlling the delivery of stimulation therapy and may further include RAM or ROM memory, logic and timing circuitry, state machine circuitry, and I/O circuitry. Typically, the microcontroller 260 includes the ability to process or monitor input signals (data) as controlled by a program code stored in a designated block of memory. The details of the design and operation of the microcontroller 260 are not critical to the present disclosure. Rather, any suitable microcontroller 260 may be used that carries out the functions described herein.

A pulse generator 270 generates pacing stimulation pulses for delivery via an electrode configuration switch 274 (for an ICD embodiment). The pulse generator 270 may include an independent pulse generator, a multiplexed pulse generator, or a shared pulse generator. The pulse generator 270 is controlled by the microcontroller 260 via an appropriate control signal to trigger or inhibit the stimulation pulses. The microcontroller 260 further includes timing control circuitry 279, which is used to control the timing of such stimulation pulses (e.g., pacing rate) as well as to keep track of the timing of refractory periods, blanking intervals, noise detection windows, evoked response windows, alert intervals, marker channel timing, etc., which is well known in the art.

The timing control circuitry 279 also controls the onset and duration of an LBB signal sensing window, during which a depolarization signal conducted through the AV node to the LBB can be detected. Timing control circuitry 279 also controls a timing delay (AV delay) provided after a detected atrial signal detection or paced event, prior to the delivery of a LBB area stimulation pulse.

The switch 274 typically includes a plurality of switches for connecting the desired electrodes to the appropriate I/O circuits, thereby providing complete electrode programmability. Accordingly, the switch 274, in response to a control signal 280 from the microcontroller 260, determines the polarity of the stimulation pulses (e.g., unipolar, bipolar, cross-chamber, etc.) by selectively closing the appropriate combination of switches (not shown) as is known in the art.

Atrial sensing circuits and ventricular sensing circuits 230 may also be selectively coupled to various electrodes through the switch 274 for detecting the presence of cardiac activity along select sensing vectors. The electrodes may be distributed between the various leads positioned to different locations in or along the heart. As explained herein, electrodes are configured to be located proximate to the LBB area. The sensing circuits are configured to sense CA signals over corresponding sensing channels associated with a corresponding port in a header of the IMD.

The outputs of the sensing circuits 230 (including the outputs of the lead tip electrodes 222 and 232) are connected to the microcontroller 260 which, in turn, is able to trigger or inhibit the pulse generator 270 in a demand fashion in response to the absence or presence of cardiac activity in the appropriate chambers of the heart. The sensing circuits 230, in turn, receive control signals from the microcontroller 260 for purposes of controlling the gain, threshold, polarization charge removal circuitry (not shown), and the timing of any blocking circuitry (not shown) coupled to the inputs of the sensing circuits.

According to one implementation, an LBB sensing circuit 230 is selectively coupled to the lead 224 for detecting the presence of a conducted depolarization arising in the atria and conducted through the LBB via the AV node. As used herein, each of the sensing circuits 230 may include a discriminator, which is a circuit that senses and can indicate or discriminate the origin of a cardiac signal in each of the cardiac chambers. The LBB sensing circuit 230 may be a dedicated circuit within the IMD 210, or LBB-related functionality may instead be provided by repurposing other pacing and sensing channels and circuitry of the IMD 210.

The IMD may have an atrial sensing circuit, a ventricular sensing circuit and an LBB sensing circuit, or a sensing circuit 230 capable of sensing cardiac activity in an atrium, in a ventricle ad through the LBB depending on which electrodes the sensing circuit 230 is selectively coupled through the switch 274.

For arrhythmia detection, the IMD 210 includes an arrhythmia detector 277 that utilizes the sensing circuits 230 to sense cardiac signals to determine whether a rhythm is physiologic or pathologic. As used herein "sensing" is reserved for the noting of an electrical signal, and "detection" is the processing of these sensed signals and noting the presence of an arrhythmia. The timing intervals between sensed events (e.g., P-waves, R-waves, and depolarization signals associated with fibrillation) are then classified by the microcontroller 260 in order to determine the type of remedial therapy that is needed. In one example, the sensing circuits 230 can be utilized to determine an RAs event and an RVs event. The RAs event may be a time at which a CA signal first reaches a threshold, such as a threshold amplitude that indicates a CA signal has been sensed. The IMD 210 may use that time as the RAs event, which is the start of the RAs,p-RVs interval. In one example, once the CA signal reaches a threshold indicating that the CA signal has reached the RV, the IMD 210 (or microcontroller 260 thereof) starts a timer set to a determined period of time. Then, a sensing circuit 230 of the IMD 210 may be utilized to detect the maximum amplitude of the CA signal during the determined period of time. The IMD 210 may use the time, at which the maximum amplitude occurs as an RVs event to determine the RAs,p-RVs interval. For example, the RVs event is the end of the RAs,p-RVs interval. The AVD may be calculated by the IMD 210 based on the RAs,p-RVs interval, such as by applying an offset to the RAs,p-RVs interval.

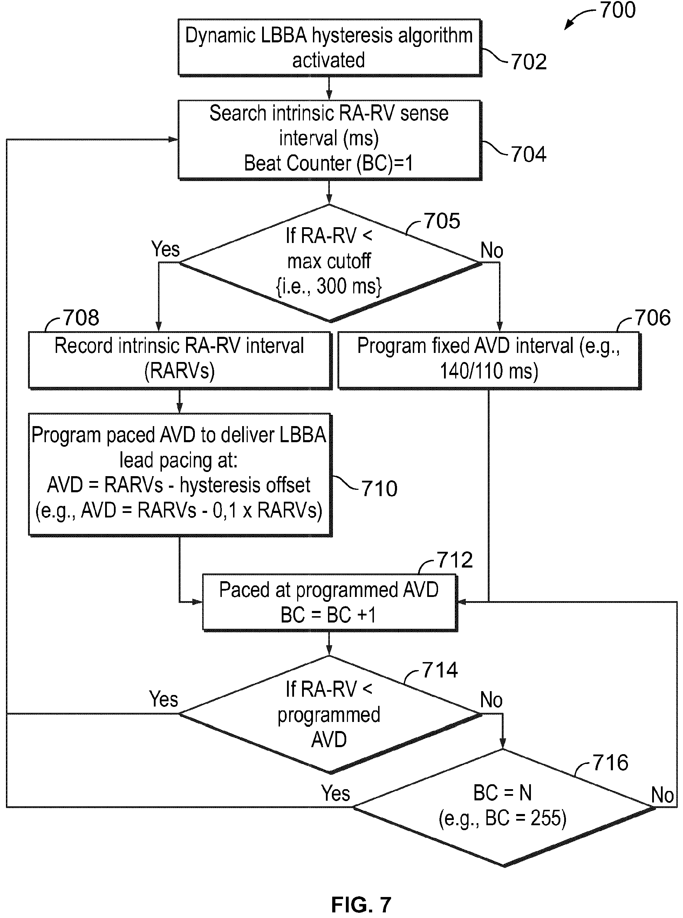

The programmable microcontroller 260 may include an AVD manager 265 that is configured to implement operations described herein, including at least some of the operations described in connection with FIG. 7. For example, the programmable microcontroller 260 may represent one or more processors that are configured to execute program instructions to implement the AVD manager 265 to determine a calculated AVD for the implantable IMD 210 as described herein. The programmable microcontroller 260 and the various circuits and modules associated therewith, including treatment circuitry 264, are configured to manage the pacing therapy based on a determined RAs,p-RVs interval.

Once a determination is made that remedial treatment is desired, the treatment circuitry 264 can determine the treatment(s) to provide. In one example, the treatment provided can be pacing at a time that results in fusion pacing (e.g., fusion between the paced LBB stimulus and an intrinsic wavefront propagating along the RBB). The fusion pacing may be achieved by determining an AVD by measuring the time delay between an RAs,p event and a RVs event (e.g., the RAs,p-RVs interval) as described. The treatment circuitry 264 can receive and/or detect CA signals by monitoring the heart and/or obtaining the CA signals from a data storage device.

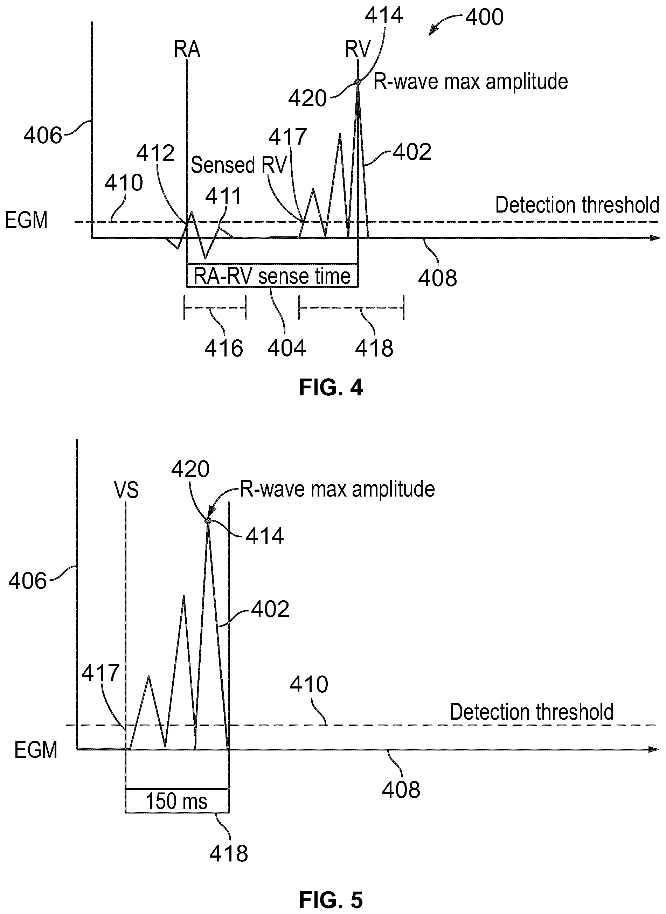

Figure 4 illustrates a graph 400 of a CA signal 402 during a single heartbeat. In one example, the CA signal is a myocardial electrical signal. In particular, the treatment circuitry 264 can analyze the CA signals on a beat-by-beat basis to determine an RAs,p-RVs interval 404 for a beat. The graph 400 illustrates voltage 406 (in millivolts (mV)) measured over time 408 (in milliseconds (ms)). A determined threshold amplitude 410 may be utilized to determine both an RAs event 412 that starts the RAs,p-RVs interval 404 and an RVs event 414 that ends the RAs,p-RVs interval 404. In another example the RAs event 412 can have a first, or RAs, threshold, and the RVs event 414 can have a second, or RVs, threshold that is different than the first threshold. In addition, there can be different thresholds based on the type of IMD. In one example, the threshold amplitude 410 can be an RAs threshold for a pacemaker where such an RAs threshold is 0.1 mV, 1 mV, 2 mV, 5 mV, or the like. In another example, an RAs threshold for a defibrillator can be 0.2 mV, 0.5 mV, 1 mV, or the like. In other examples, the RVs threshold for a pacemaker may be 0.5 mV, 2 mV, 5 mV, 10 mV, 12 mV, etc., and the RVs threshold for a defibrillator may be 0.2 mV, 0.5 mV, 1 mV, 2 mV, etc.

In one example, the RAs event 412 occurs when the threshold amplitude 410 is first reached (e.g., crossed) by the CA signal. The RAs event 412 begins the measurement of the RAs,p-RVs interval 404. In one example, the threshold amplitude 410 can be determined such that if additional peaks 411 related to the RAs are detected, such detected additional peaks 411 may have lower amplitudes than the threshold amplitude 410. Alternatively, in another example, after the CA signal 402 initially reaches the threshold amplitude 410, an RA determined period of time 416 (e.g., blackout period) may be provided before any additional readings are detected. The RA determined period of time can be 10 ms, 20 ms, 50 ms, or the like. The RA determined period of time 416 may ensure that RAs signals are not detected and considered RVs signals.

After the RAs or RAp event is detected, the RVs event is then detected as illustrated in FIG. 5. Figure 5 illustrates an enlarged portion of the graph 400 shown in Figure 4. In an example, the RVs event 414 does not coincide with the CA signal initially reaching (e.g., crossing) a threshold amplitude. Instead, upon detecting that the CA signal 402 reaches the threshold amplitude at point 417, the IMD 210 triggers the start (VS) of an RVs determined period of time 418. In example embodiments, the RVs determined period of time 418 can be 120 ms, 150 ms, 180 ms, or the like. In Figure 5, the determined period of time is shown as 150 ms. During the RVs determined period of time 418, the sensing circuitry 230 and/or treatment circuitry 264 continues to monitor the CA signal 402 to determine the peak amplitude 420 during the RVs determined period of time 418. The treatment circuitry 264 then utilizes the peak amplitude 420 as the RVs event 414 (e.g., the end of the RAs,p-RVs interval 404). Thus, in a first example, the RAs,p-RVs interval 404 may be measured from when the CA signal 402 first reaches the threshold amplitude 410 to the time at which the CA signal 402 achieves a peak amplitude 420 within the RVs determined period of time after surpassing the threshold amplitude 410 associated with the RV. In a second example, the RAs,p-RVs interval 404 may be measured from when there is a paced atrial event (e.g., RAp event) to the time at which the CA signal 402 achieves the peak amplitude 420 within the RVs determined period of time after surpassing the threshold amplitude 410 associated with the RV.

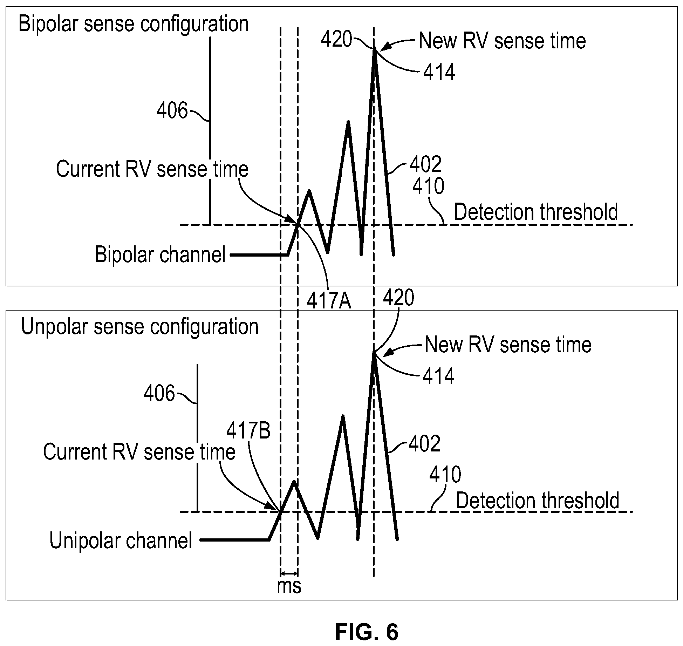

FIG. 6 illustrates the advantage of utilizing the peak amplitude 420 detected during the RVs determined period of time 418 as the RVs event 414. In particular, when a bipolar sense configuration (top panel of FIG. 6) is utilized by the IMD 210, the CA signal 402 reaches the threshold amplitude 410 at a first time 417A. In contrast, when a unipolar sense configuration (bottom panel of FIG. 6) is utilized by the IMD 210, the CA signal may reach the threshold amplitude 410 at a second time 417B that is different than the first time 417A. By using the RVs determined period of time 418 and determining the peak amplitude 420 reached within the RVs determined period of time 418, both unipolar and bipolar sense configurations provide the same RVs event 414, and also the same RAs,p-RVs interval 404 (as shown in Figure 4). Consequently, more accurate and consistent results are obtained utilizing this methodology.

With reference back to FIG. 3, once the RAs,p-RVs interval is determined, the microcontroller 260 (e.g., the treatment circuitry 264) preferably utilizes an offset to determine the AVD. In one example, the offset is 10% of the measured RAs,p-RVs interval. Alternatively, the offset can be 8 %, 12 %, 15 %, or the like, of the measured RAs,p-RVs interval. To this end, the offset can be subtracted from the RAs,p-RVs interval to calculate the AVD. The treatment circuitry 264 may then utilize the calculated AVD to manage and provide pacing therapy. In particular, the treatment circuitry 264 can be utilized to manage pacing therapy, used by the IMD 210, based on the calculated AVD. In one example, to manage the pacing therapy, the ventricular tip electrode 232 of the lead 224 delivers LBB area pacing therapy based on the AVD. As a result of using the methodology, fusion pacing may be accomplished.

The microcontroller 260 (e.g., the treatment circuitry 264) may continue to detect the RAs,p-RVs interval over time. For example, if the RAs,p-RVs interval at a future time is detected to be less than the AVD, the microcontroller 260 may restart the process to determine an updated AVD. In particular, the RAs,p-RVs interval of a patient can change over time as a result of patient activity. Such patient activity can include exercising, stressful situations, mode swings, diet, or the like. The change can result in the AVD no longer successfully achieving fusion pacing. Thus, the microcontroller 260 may continuously monitor to ensure that the RAs,p-RVs interval has not significantly changed. In one example, a significant change may be detected when a measured RAs,p-RVs interval is less than the AVD. Because an offset has been subtracted from the RAs,p-RVs interval in determining the AV delay, the measured RAs,p-RVs interval should not be less than the AVD. If this situation is detected, the microcontroller 260 may update the AVD. Thus, if the RAs,p-RVs changes as a result of exercising, excitement, stress, other stimulus, or the like, the RAs,p-RVs is updated accordingly. Similarly, the treatment circuitry 264 may automatically redetermine the AVD after a designated determined number of CA signals. In this manner, the AVD may be repeatably updated over time and/or adjusted based on the activity of the patient. This dynamic adjustment improves treatment for the patient.

Cardiac signals are preferably also applied to the inputs of an analog-to-digital (A/D) data acquisition system (DAS) 290. The A/D data acquisition system 290 may include an A/D converter. The data acquisition system 290 is configured to acquire intracardiac electrogram signals (which represent raw analog data), convert the raw analog data into digital signals, and store the digital signals for later processing and/or telemetric transmission to an external device 293. In one example, the data acquisition system 290 is coupled to microcontroller 260, or to other detection circuitry, for detecting a desired feature of the HIS bundle signal.

Advantageously, the data acquisition system 290 may detect an evoked response from the heart 212 in response to an applied stimulus, thereby aiding in the detection of capture. The microcontroller 260 detects a depolarization signal during a window following a stimulation pulse, the presence of which indicates that capture has occurred. The microcontroller 260 is further coupled to a memory 294 by a suitable data/address bus 296. The memory 294 may store programmable operating parameters used by the microcontroller 260. The operating parameters may be modified, as required, in order to customize the operation of the IMD 210 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart 212 within each respective tier of therapy.

Advantageously, the operating parameters of the IMD 210 may be non-invasively programmed into the memory 294 through a telemetry circuit 266 in telemetric communication with the external device 293, such as a programmer, trans-telephonic transceiver, or a diagnostic system analyzer. The telemetry circuit 266 is activated by the microcontroller 260 by a control signal. The telemetry circuit 266 advantageously allows intracardiac electrograms and status information relating to the operation of the IMD 210 (as contained in the microcontroller 260 or memory 294) to be sent to the external device 293 through an established communication link 295.

In certain implementations, the IMD 210 may further include a physiologic sensor 299, such as a "rate-responsive" sensor, to adjust pacing stimulation rate based on the exercise state of the patient. Optionally, the physiological sensor 299 may be used to detect changes in cardiac output, changes in the physiological condition of the heart, or diurnal changes in activity (e.g., detecting sleep and wake states). Accordingly, the microcontroller 260 responds by adjusting the various pacing parameters (such as rate, stimulation delays, etc.) at which the pulse generator 270 generates stimulation pulses.

The IMD 210 additionally includes a battery 291 which provides operating power to all of the circuits and electrical components shown in Figure 3. For the IMD 210, which can provide shocking therapy, the battery 291 is capable of operating at low current drains for long periods of time, and then providing high-current pulses (for capacitor charging) when the patient requires a shock pulse. The battery 291 may have a predictable discharge characteristic so that elective replacement time can be detected. The battery 291 may include one or more lithium/silver vanadium oxide battery cells.

The IMD 210 may have an impedance measuring circuit 312, which is enabled by the microcontroller 260 via a control signal. The impedance measuring circuit 312 may perform lead impedance surveillance during acute and chronic phases for detecting proper lead positioning or dislodgement, may detect operable electrodes and conductors, and/or may automatically switch to an operable pair if dislodgement or electrical disruption occurs. The impedance measuring circuit 312 may measure respiration or minute ventilation, thoracic impedance for determining shock thresholds, and/or stroke volume. The impedance measuring circuit 312 may detect when the IMD 210 has been implanted and/or the opening of heart valves, etc. The impedance measuring circuit 312 is advantageously coupled to the switch 274 so that any desired electrode may be used.

In an example in which the IMD 210 is operable as an implantable cardioverter/defibrillator (ICD) device, in response to detecting an arrhythmia, the IMD 210 may automatically apply an appropriate electrical shock therapy to the heart aimed at terminating the detected arrhythmia. To this end, the microcontroller 260 further controls a shocking circuit 316 by way of a control signal. The shocking circuit 316 generates shocking pulses of low (for example, up to 0.5 joules), moderate (for example, 0.5-10 joules), or high energy (for example, 11-40 joules), as controlled by the microcontroller 260. Such shocking pulses are applied to the patient's heart 212 through shocking electrodes. As noted above, the housing 240 may act as an active electrode in combination with other electrodes.

Cardioversion shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 5-40 joules), delivered asynchronously (since R-waves may be too disorganized), and pertaining exclusively to the treatment of fibrillation. Accordingly, the microcontroller 260 is capable of controlling the synchronous and/or asynchronous delivery of the shocking pulses.

### Dual Chamber Pacing with LBB Electrode

Next, a configuration is described in connection with an IMD configured to provide dual chamber pacing utilizing an electrode located proximate to the LBB. The electrode may be connected to an RV port in the IMD.

Figure 7 illustrates a non-claimed process 700 (e.g., method) for managing stimulation therapy of an IMD in accordance with embodiments herein. The IMD may have the electrode configuration shown in Figure 2. In one example, the process 700 may be implemented utilizing one or more processors of the IMD, such as the microcontroller 260 in Fig. 3. The one or more processors may function within the treatment circuitry 264 as provided in Fig. 3 to accomplish the process 700.

At step 702, the one or more processors activate a dynamic pacing management algorithm. In one example, the pacing management algorithm includes determining an AVD by using the RAs,p-RVs interval determined as described in relation to FIGS. 4-6. In an example, the RAs event is defined as the time at which a CA signal initially crosses a threshold amplitude. The RVs event is defined as the time of a peak amplitude of the CA signal detected during an RV determined period of time after the CA signal reaches a threshold associated with an RV period. The RAp event is defined as the time at which a pacing pulse is delivered by an implantable lead. The AVD is then determined based on the time interval (e.g., delay) between the RAs or RAp event and the RVs event (e.g., the RAs,p-RVs interval).

At step 704, the one or more processors monitor and obtain CA signals for detecting COls of the CA signals. In one example, the CA signals are myocardial electrical signals that are monitored on a beat-by-beat basis. In another example, the COls include RA COls including RAs events, RV COls including RVs events, and/or an RAs,p-RVs interval. In addition, a beat counter is provided that registers the heartbeat that includes the CA signal as a first beat, or a counted CA signal. A determination can be made at step 705 whether a determined period of time has elapsed since last determining the RAs,p-RVs interval. If the heart of the patient stops beating, then CA signals cannot be obtained at 704, so the determined period of time can be used as a check on whether the heart of the patient is still beating. If the heart of the patient is not beating, regardless of the steps in this method, a pace needs to be provided to the patient. In one example, the determined period of time can be 300 ms. The one or more processors determine if an intrinsic CA signal can be detected (e.g., whether the heart of the patient is beating). If no intrinsic CA signal is detected, then at step 706, a fixed AVD is utilized for pacing until the heart of the patient begins working again and producing CA signals. The fixed AVD may be a preset, default AVD value. In particular, after the IMD is implanted, a first intrinsic heartbeat needs to occur in order to measure the RAs,p-RVs interval as described to dynamically determine the AVD interval. Typically that first heartbeat occurs such that the fixed AVD never needs to be utilized. However, if the heart does not provide an intrinsic CA signal for a potential beat, the fixed AVD is provided as a safeguard for pacing. The fixed AVD may be used until a CA signal is detected. The fixed AVD interval can be 25 ms, 50 ms, 80 ms, 100 ms, 110 ms, 140 ms, 150 ms, etc.

If the intrinsic CA signal is detected at step 705, then at step 708 the one or more processors of the IMD determine the RAs,p-RVs interval. As indicated, in one example the RAs,p-RVs interval is determined by identifying the RAs event (or RAp event) and RVs event as endpoints of the interval as described in relation to FIGs. 4-6. The RAs,p-RVs interval may be measured and recorded. By using this technique, or methodology, errors related to the utilization of bipolar or unipolar configurations can be reduced or eliminated.

At step 710, the one or more processors determine the AVD to use for pacing via at least one of the leads. In one example, the AVD is calculated by subtracting a hysteresis offset from the determined RAs,p-RVs interval (e.g., AVD = RAs,p-RVs interval - offset). In one example, the offset can be a determined amount of offset such as 10 ms, 12 ms, 15 ms, etc. Alternatively, the hysteresis offset may be a percentage of the RAs,p-RVs interval such as 10%, 12%, 15%, or the like. In an example in which the offset is 10% of the interval, the offset may be calculated as 0.1 x the RAs,p-RVs interval value. By utilizing the hysteresis offset, the timing of pacing can be improved to achieve fusion pacing.

At step 712, the one or more processors deliever therapy to the heart by pacing the patient heart based on either the fixed AVD value at step 706 (until an intrinsic CA signal is detected) or the calculated AVD from step 710. The beat counter may be incrementally updated for each intrinsic beat that is detected to increase the number of counted CA signals. In general, patients alter their activity levels over time, which results in CA signals with varying characteristics of interest. For example, patients can exercise, become sick, become stressed, change emotions, sleep, or the like, all of which can result in morphological changes in CA signal on a beat-to-beat basis. For example, the CA signals during a jog may have significantly different COIs compared to COls when the same person is sitting, sleeping, or relaxing. The process 700 may include verification steps (714 and 716) to update the determined AVD at step 710 over time. The process 700 may program the IMD 210 to deliver pacing therapy using the calculated AVD as a pacing parameter.

At step 714, the one or more processors continue to repeatedly and continuously determine the RAs,p-RVs interval for each beat and determine whether the RAs,p-RVs interval is less than the AVD. Because the calculated (and programmed) AVD incorporates the hysteresis offset subtracted from the RAs,p-RVs interval, the RAs,p-RVs is expected to be greater (e.g., longer) than the calculated AVD. If an updated RAs,p-RVs interval is determined to be less (e.g., shorter) than the programmed AVD, the process 700 may determine that a reset is needed. The RAs,p-RVs interval may change as a result of an exterior event such as jogging, walking, mode change, or the like. Thus, if the RAs,p-RVs interval is found to be less than the programmed AVD, the system begins searching for the next intristic CA signal to provide a new calculation for the AVD to utilize for managing the pacing therapy. The programmed AVD is dynamically updated to improve treatment.

Alternatively, or in addition, at step 716, if the RAs,p-RVs interval is not smaller than the programmed AVD, then the one or more processors determine whether the number of counted CA signals have reached a determined number of CA signals. If the determined number of CA signals is not reached, the IMD continues pacing using the calculated AVD. If the determined number of CA signals is reached, then the IMD restarts the process by searching for an intrinsic CA signal at step 704 for determining a new AVD value.

The IMD operates to automatically reset after a determined number of CA signals (e.g., number of counted CA signals, as measured in beats) to improve the accuracy of the calculated AVD value. In one example, the determined number of counted CA signals can be 255 beats. In other examples, the determined number of counted CA signals can be 225 beats, 250 beats, 275 beats, 310 beats, or the like. The IMD dynamically updates the calculated AVD value periodically in real time, so that the therapy can be adjusted while the patient partakes in different activites, has changing emotions and moods, or the like. For example, if the patient has a pulse rate of 60 beats per minute, and the determined number of counted CA signals is 240 beats, then the IMD calculates a new AVD value every 4 minutes for use with pacing. The dynamic updating ensures that the AVD value changes with changes in patient activities, moods, etc. to improve treatment through pacing. In particular, by dynamically updating the AVD value, fusion pacing may be accomplished more often than if the process 700 is not utlized, resulting in narrowing of the QRS duration and improved health.

In accordance with examples here a non-claimed method for managing therapy can include determining, with one or more processors of an implantable medical device (IMD), a sensed right atrium (RAs) event or a paced right atrium (RAp) event (RAs,p event) of a cardiac activity (CA) signal and determining, with the one or more processors of the IMD, a sensed right ventricle (RVs) event of the cardiac activity (CA) signal by detecting the CA signal reaches a threshold amplitude and determining a maximum amplitude in a determined period of time after the CA signal reaches the threshold amplitude. The method can also include determining, with the one or more processors of the IMD, an RAs,p-RVs interval between the RAs,p event and RVs event, calculating, with the one or more processors of the IMD, an atrioventricular delay (AV delay) based on the RAs,p-RVs interval, and managing therapy, provided by the IMD, based on the AV delay calculated.

In one example, managing the therapy may include delivering, with an electrode of the IMD, pacing therapy based on the AV delay to a left bundle branch (LLB) area of a heart.

In one example, calculating the AV delay can include reducing the RAs,p-RVs interval by a determined percentage of the RAs,p-RVs interval.

In one example, the method can additionally include, after calculating the AV delay, setting, with the one or more processors of the IMD, the AV delay as an initial AV delay and utilizing, with the one or more processors, the initial AV delay to manage the therapy for a determined number of CA signals.

In one example, the method can also include counting, with the one or more processors of the IMD, a counted number of the CA signals and comparing, with the one or more processors of the IMD, the counted number of CA signals to the determined number of CA signals. The method can also include repeating, with the one or more processors of the IMD, each of the determining steps and the calculating step to calculate an updated AV delay after the determined number of CA signals obtained is reached and utilizing, with the one or more processors, the updated AV delay to manage the therapy.

In one example, the method may also include resetting, with the one or more processors of the IMD, the counted CA signals in response to calculating the updated AV delay.

In one example, the method can additionally include measuring a current RAs,p-RVs interval for a current CA signal and repeating, with the one or more processors of the IMD, each of the determining steps and the calculating step to calculate an updated AV delay in response to the current RAs,p-RVs interval being less than the AV delay.

In one example, the method can also include counting, with the one or more processors, a number of counted CA signals and comparing, with the one or more processors of the IMD, the number of counted CA signals to a determined number of CA signals. The method may additionally include resetting, with the one or more processors of the IMD, the number of counted CA signals in response to calculating the updated AV delay.

In one example, the method can additionally include delivering, with an electrode of the IMD, left bundle block area pacing therapy based on the updated AV delay.

### CLOSING

The various non-claimed methods as illustrated in the Figures and described herein represent exemplary embodiments of methods. The methods may be implemented in software, hardware, or a combination thereof. In various of the methods, the order of the steps may be changed, and various elements may be added, reordered, combined, omitted, modified, etc. Various steps may be performed automatically (e.g., without being directly prompted by user input) and/or programmatically (e.g., according to program instructions).

Various modifications and changes may be made as would be obvious to a person skilled in the art having the benefit of this disclosure. It is intended to embrace all such modifications and changes and, accordingly, the above description is to be regarded in an illustrative rather than a restrictive sense.

Various embodiments of the present disclosure utilize at least one network that would be familiar to those skilled in the art for supporting communications using any of a variety of commercially-available protocols, such as Transmission Control Protocol/Internet Protocol ("TCP/IP"), User Datagram Protocol ("UDP"), protocols operating in various layers of the Open System Interconnection ("OSI") model, File Transfer Protocol ("FTP"), Universal Plug and Play ("UpnP"), Network File System ("NFS"), Common Internet File System ("CIFS") and AppleTalk. The network can be, for example, a local area network, a wide-area network, a virtual private network, the Internet, an intranet, an extranet, a public switched telephone network, an infrared network, a wireless network, a satellite network and any combination thereof.

In embodiments utilizing a web server, the web server can run any of a variety of server or mid-tier applications, including Hypertext Transfer Protocol ("HTTP") servers, FTP servers, Common Gateway Interface ("CGI") servers, data servers, Java servers, Apache servers and business application servers. The server(s) also may be capable of executing programs or scripts in response to requests from user devices, such as by executing one or more web applications that may be implemented as one or more scripts or programs written in any programming language, such as Java^{®}, C, C# or C++, or any scripting language, such as Ruby, PHP, Perl, Python or TCL, as well as combinations thereof. The server(s) may also include database servers, including without limitation those commercially available from Oracle^{®}, Microsoft^{®}, Sybase^{®} and IBM^{®} as well as open-source servers such as MySQL, Postgres, SQLite, MongoDB, and any other server capable of storing, retrieving and accessing structured or unstructured data. Database servers may include table-based servers, document-based servers, unstructured servers, relational servers, non-relational servers or combinations of these and/or other database servers.

The environment can include a variety of data stores and other memory and storage media as discussed above. These can reside in a variety of locations, such as on a storage medium local to (and/or resident in) one or more of the computers or remote from any or all of the computers across the network. In a particular set of embodiments, the information may reside in a storage-area network ("SAN") familiar to those skilled in the art. Similarly, any necessary files for performing the functions attributed to the computers, servers or other network devices may be stored locally and/or remotely, as appropriate. Where a system includes computerized devices, each such device can include hardware elements that may be electrically coupled via a bus, the elements including, for example, at least one central processing unit ("CPU" or "processor"), at least one input device (e.g., a mouse, keyboard, controller, touch screen or keypad) and at least one output device (e.g., a display device, printer or speaker). Such a system may also include one or more storage devices, such as disk drives, optical storage devices and solid-state storage devices such as random-access memory ("RAM") or read-only memory ("ROM"), as well as removable media devices, memory cards, flash cards, etc.

Such devices also can include a computer-readable storage media reader, a communications device (e.g., a modem, a network card (wireless or wired), an infrared communication device, etc.) and working memory as described above. The computer-readable storage media reader can be connected with, or configured to receive, a computer-readable storage medium, representing remote, local, fixed and/or removable storage devices as well as storage media for temporarily and/or more permanently containing, storing, transmitting and retrieving computer-readable information. The system and various devices also typically will include a number of software applications, modules, services or other elements located within at least one working memory device, including an operating system and application programs, such as a client application or web browser. It should be appreciated that alternate embodiments may have numerous variations from that described above. For example, customized hardware might also be used and/or particular elements might be implemented in hardware, software (including portable software, such as applets) or both. Further, connection to other computing devices such as network input/output devices may be employed.

Various embodiments may further include receiving, sending, or storing instructions and/or data implemented in accordance with the foregoing description upon a computer-readable medium. Storage media and computer readable media for containing code, or portions of code, can include any appropriate media known or used in the art, including storage media and communication media, such as, but not limited to, volatile and non-volatile, removable and non-removable media implemented in any method or technology for storage and/or transmission of information such as computer readable instructions, data structures, program modules or other data, including RAM, ROM, Electrically Erasable Programmable Read-Only Memory ("EEPROM"), flash memory or other memory technology, Compact Disc Read-Only Memory ("CD-ROM"), digital versatile disk (DVD) or other optical storage, magnetic cassettes, magnetic tape, magnetic disk storage or other magnetic storage devices or any other medium which can be used to store the desired information and which can be accessed by the system device. Based on the disclosure and teachings provided herein, a person of ordinary skill in the art will appreciate other ways and/or methods to implement the various embodiments.

The specification and drawings are, accordingly, to be regarded in an illustrative rather than a restrictive sense. It will, however, be evident that various modifications and changes may be made thereunto without departing from the scope of the invention as set forth in the claims.

Other variations are within the scope of the present disclosure. Thus, while the disclosed techniques are susceptible to various modifications and alternative constructions, certain illustrated embodiments thereof are shown in the drawings and have been described above in detail. It should be understood, however, that there is no intention to limit the invention to the specific form or forms disclosed.

The use of the terms "a" and "an" and "the" and similar referents in the context of describing the disclosed embodiments (especially in the context of the following claims) are to be construed to cover both the singular and the plural, unless otherwise indicated herein or clearly contradicted by context. The terms "comprising," "having," "including" and "containing" are to be construed as open-ended terms (i.e., meaning "including, but not limited to,") unless otherwise noted. The term "connected," when unmodified and referring to physical connections, is to be construed as partly or wholly contained within, attached to or joined together, even if there is something intervening. Recitation of ranges of values herein are merely intended to serve as a shorthand method of referring individually to each separate value falling within the range, unless otherwise indicated herein and each separate value is incorporated into the specification as if it were individually recited herein. The use of the term "set" (e.g., "a set of items") or "subset" unless otherwise noted or contradicted by context, is to be construed as a nonempty collection comprising one or more members. Further, unless otherwise noted or contradicted by context, the term "subset" of a corresponding set does not necessarily denote a proper subset of the corresponding set, but the subset and the corresponding set may be equal.

Operations of processes described herein can be performed in any suitable order unless otherwise indicated herein or otherwise clearly contradicted by context. Processes described herein (or variations and/or combinations thereof) may be performed under the control of one or more computer systems configured with executable instructions and may be implemented as code (e.g., executable instructions, one or more computer programs or one or more applications) executing collectively on one or more processors, by hardware or combinations thereof. The code may be stored on a computer-readable storage medium, for example, in the form of a computer program comprising a plurality of instructions executable by one or more processors. The computer-readable storage medium may be non-transitory.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the invention without departing from its scope. While the dimensions, types of materials and physical characteristics described herein are intended to define the parameters of the invention, they are by no means limiting and are exemplary embodiments. Many other embodiments will be apparent to those of skill in the art upon reviewing the above description. The scope of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An implantable medical device (IMD) (210) for managing therapy comprising:
a connector coupleable to a lead (220, 224) with an electrode (222, 232);
a memory (294) configured to store program instructions; and
one or more processors (260) configured to execute the program instructions to:
determine a sensed right atrium (RAs) event or a paced right atrial (RAp) event (RAs,p event) (412);
determine a sensed right ventricle (RVs) event (414) by detecting a cardiac activity (CA) signal (402) reaches a threshold amplitude (410) and determine a maximum amplitude (420) in a determined period of time (418) after the CA signal (402) reaches the threshold amplitude (410);
determine an RAs,p-RVs interval (404) between the RAs,p event (412) and RVs event (414);
calculate an atrioventricular delay (AV) delay based on the RAs,p-RVs interval (404); and
manage therapy, provided by the IMD (210), based on the AV delay that is calculated.

2. The IMD of claim 1, wherein the one or more processors (260) are configured to manage the therapy by controlling the electrode (222, 232) to deliver left bundle branch area pacing therapy based on the AV delay.

3. The IMD of claim 1 or 2, wherein the IMD (210) is a dual chamber implantable pulse generator (IPG).

4. The IMD of any one of claims 1 to 3, wherein the one or more processors (260) are configured to calculate the AV delay by subtracting a determined percentage of the RAs,p-RVs interval (404) from the RAs,p-RVs interval (404).

5. The IMD of any one of claims 1 to 4, wherein the CA signal (402) is an intrinsic CA signal (402).

6. The IMD of any one of claims 1 to 5, wherein the one or more processors (260) are further configured to:
after calculating the AV delay, set the AV delay as an initial AV delay; and
utilize the initial AV delay to manage the therapy for a determined number of CA signals.

7. The IMD of claim 6, wherein the one or more processors (260) are further configured to:
count a number of the CA signals after a first CA signal;
compare a number of counted CA signals to the determined number of CA signals;
repeat determination of the RAs,p event (412), determination of the RVs event (414), determination of the RAs,p-RVs interval (404) and calculation of the AV delay after the number of counted CA signals matches the determined number of CA signals, to calculate an updated AV delay; and
manage the therapy provided by the IMD (210), based on the updated AV delay.

8. The IMD of claim 7, wherein the one or more processors (260) are further configured to reset a count of the number of counted CA signals in response to calculating the updated AV delay.

9. The IMD of any one of claims 1 to 7, wherein the one or more processors (260) are further configured to:
after calculating the AV delay, determine a second RAs,p-RVs interval based on a second CA signal,
compare the second RAs,p-RVs interval to the AV delay; and
responsive to the second RAs,p-RVs interval being less than the AV delay, calculate an updated AV delay based on the second RAs,p-RVs interval.

10. The IMD of claim 9, wherein the one or more processors (260) are further configured to:
count a number of CA signals obtained after a first CA signal,
compare a counted number of CA signals to a determined number of CA signals; and
reset the counted number of the CA signals in response to calculating the updated AV delay.

11. The IMD of claim 9 or 10, wherein the one or more processors (260) are further configured to manage the therapy, provided by the IMD (210), based on the updated AV delay, wherein the one or more processors (260) are configured to manage the therapy by controlling the electrode (222, 224) to deliver left bundle branch area pacing therapy based on the updated AV delay.

12. The IMD of any one of claims 1 to 11, further comprising a sensing circuitry (230) configured to be coupled to the electrode (222, 232) to detect the CA signal (402).

13. The IMD of any one of claims 1 to 12, further comprising a pulse generator (270) controlled by the one or more processors (260) to deliver pacing stimulation pulses via the electrode (222, 232).

14. The IMD of claim 13, wherein the one or more processors (260) are further configured to control the pulse generator (270) to deliver a pacing stimulation pulse via the electrode (222, 232) at the expiry of the AV delay.

15. The IMD of any one of claims 1 to 14, wherein the one or more processors (260) are configured to determine the RVs event (414) as the maximum amplitude (420) in the CA signal (402) in the determined period of time (418) after the CA signal (402) reaches the threshold amplitude (410).

## Patentansprüche

1. Implantierbare medizinische Vorrichtung (IMD) (210) zum Verwalten einer Therapie, umfassend:
einen Verbinder, der an eine Leitung (220, 224) mit einer Elektrode (222, 232) koppelbar ist;
einen Speicher (294), der dazu konfiguriert ist, Programmanweisungen zu speichern; und
einen oder mehrere Prozessoren (260), die dazu konfiguriert sind, die Programmanweisungen zu Folgendem auszuführen:
Bestimmen eines erfassten Ereignisses im rechten Vorhof (RAs) oder eines regulierten Ereignisses im rechten Vorhof (RAp) (RAs,p-Ereignis, 412);
Bestimmen eines erfassten Ereignisses (414) im rechten Ventrikel (RVs) durch Erkennen, dass ein Herzaktivitätssignal (CA-Signal) (402) eine Schwellenamplitude (410) erreicht, und Bestimmen, dass eine maximale Amplitude (420) in einem bestimmten Zeitraum (418) nach dem CA-Signal (402) die Schwellenamplitude (410) erreicht;
Bestimmen eines RAs,p-RVs-Intervalls (404) zwischen dem RAs,p-Ereignis (412) und dem RVs-Ereignis (414);
Berechnen einer atrioventrikulären Verzögerung Verzögerung (AV-Verzögerung) basierend auf dem RAs,p-RVs-Intervall (404); und
Verwalten einer Therapie, die durch das IMD (210) bereitgestellt wird, basierend auf der AV-Verzögerung, die berechnet wird.

2. IMD nach Anspruch 1, wobei der eine oder die mehreren Prozessoren (260) dazu konfiguriert sind, die Therapie basierend auf der AV-Verzögerung dadurch zu verwalten, dass die Elektrode (222, 232) dazu gesteuert wird, eine Regulierungstherapie im Bereich des Linksschenkelblocks durchzuführen.

3. IMD nach Anspruch 1 oder 2, wobei das IMD (210) ein implantierbarer Zweikammer-Impulsgenerator (Zweikammer-IPG) ist.

4. IMD nach einem der Ansprüche 1 bis 3, wobei der eine oder die mehreren Prozessoren (260) dazu konfiguriert sind, die AV-Verzögerung durch Subtrahieren eines bestimmten Prozentsatzes des RAs,p-RVs-Intervalls (404) von dem RAs,p-RVs-Intervall (404) zu berechnen.

5. IMD nach einem der Ansprüche 1 bis 4, wobei das CA-Signal (402) ein intrinsisches CA-Signal (402) ist.

6. IMD nach einem der Ansprüche 1 bis 5, wobei der eine oder die mehreren Prozessoren (260) ferner zu Folgendem konfiguriert sind:
nach dem Berechnen der AV-Verzögerung, Einstellen der AV-Verzögerung als eine anfängliche AV-Verzögerung; und
Verwenden der anfänglichen AV-Verzögerung, um die Therapie für eine bestimmte Anzahl an CA-Signalen zu verwalten.

7. IMD nach Anspruch 6, wobei der eine oder die mehreren Prozessoren (260) ferner zu Folgendem konfiguriert sind:
Zählen einer Anzahl der CA-Signale nach einem ersten CA-Signal;
Vergleichen einer Anzahl gezählter CA-Signale mit der bestimmten Anzahl an CA-Signalen;
Wiederholen der Bestimmung des RAs,p-Ereignisses (412), der Bestimmung des RVs-Ereignisses (414), der Bestimmung des RAs,p-RVs-Intervalls (404) und der Berechnung der AV-Verzögerung, nachdem die Anzahl gezählter CA-Signale der bestimmten Anzahl an CA-Signalen entspricht, um eine aktualisierte AV-Verzögerung zu berechnen; und
Verwalten der Therapie, die durch das IMD (210) bereitgestellt wird, basierend auf der aktualisierten AV-Verzögerung.

8. IMD nach Anspruch 7, wobei der eine oder die mehreren Prozessoren (260) ferner dazu konfiguriert sind, eine Zählung der Anzahl gezählter CA-Signale als Reaktion auf das Berechnen der aktualisierten AV-Verzögerung zurückzusetzen.

9. IMD nach einem der Ansprüche 1 bis 7, wobei der eine oder die mehreren Prozessoren (260) ferner zu Folgendem konfiguriert sind:
nach dem Berechnen der AV-Verzögerung, Bestimmen eines zweiten RAs,p-RVs-Intervalls basierend auf einem zweiten CA-Signal;
Vergleichen des zweiten RAs,p-RVs-Intervalls mit der AV-Verzögerung; und
als Reaktion darauf, dass das zweite RAs,p-RVs-Intervall geringer ist als die AV-Verzögerung, Berechnen einer aktualisierte AV-Verzögerung basierend auf dem zweiten RAs,p-RVs-Intervall.

10. IMD nach Anspruch 9, wobei der eine oder die mehreren Prozessoren (260) ferner zu Folgendem konfiguriert sind:
Zählen einer Anzahl von CA-Signalen, die nach einem ersten CA-Signal erhalten wurden;
Vergleichen einer gezählten Anzahl an CA-Signalen mit einer bestimmten Anzahl an CA-Signalen; und
Zurücksetzen der gezählten Anzahl der CA-Signale als Reaktion auf das Berechnen der aktualisierten AV-Verzögerung.

11. IMD nach Anspruch 9 oder 10, wobei der eine oder die mehreren Prozessoren (260) ferner dazu konfiguriert sind, die Therapie, die durch das IMD (210) bereitgestellt wird, basierend auf der aktualisierten AV-Verzögerung zu verwalten, wobei der eine oder die mehreren Prozessoren (260) dazu konfiguriert sind, die Therapie basierend auf der aktualisierten AV-Verzögerung dadurch zu verwalten, dass die Elektrode (222, 224) dazu gesteuert wird, eine Regulierungstherapie im Bereich des Linksschenkelblocks durchzuführen.

12. IMD nach einem der Ansprüche 1 bis 11, ferner umfassend eine Sensing-Schaltung (230), die dazu konfiguriert ist, an die Elektrode (222, 232) gekoppelt zu werden, um das CA-Signal (402) zu erkennen.

13. IMD nach einem der Ansprüche 1 bis 12, ferner umfassend einen Impulsgenerator (270), der von dem einen oder den mehreren Prozessoren (260) gesteuert wird, um über die Elektrode (222, 232) Regulierungsstimulationsimpulse abzugeben.

14. IMD nach Anspruch 13, wobei der eine oder die mehreren Prozessoren (260) ferner dazu konfiguriert sind, den Impulsgenerator (270) dazu zu steuern, über die Elektrode (222, 232) einen Regulierungsstimulationsimpuls bei Ablauf der AV-Verzögerung abzugeben.

15. IMD nach einem der Ansprüche 1 bis 14, wobei der eine oder die mehreren Prozessoren (260) dazu konfiguriert sind, das RVs-Ereignis (414) als die maximale Amplitude (420) in dem CA-Signal (402) in dem bestimmten Zeitraum (418), nachdem das CA-Signal (402) die Schwellenamplitude (410) erreicht, zu bestimmen.

## Revendications

1. Dispositif médical implantable (DMI) (210) pour la gestion d'une thérapie comprenant :
un connecteur pouvant être couplé à une sonde (220, 224) avec une électrode (222, 232) ;
une mémoire (294) configurée pour stocker des instructions de programme ; et
un ou plusieurs processeurs (260) configurés pour exécuter les instructions de programme pour :
déterminer un événement d'oreillette droite détecté (RAs) ou un événement d'oreillette droite stimulé (RAp) (événement RAs,p) (412) ;
déterminer un événement de ventricule droit détecté (RVs) (414) en détectant qu'un signal d'activité cardiaque (AC) (402) atteint une amplitude seuil (410) et déterminer une amplitude maximale (420) dans une période de temps déterminée (418) après que le signal d'AC (402) a atteint l'amplitude seuil (410) ;
déterminer un intervalle RAs,p-RVs (404) entre l'événement RAs,p (412) et l'événement RVs (414) ;
calculer un retard auriculo-ventriculaire (AV) sur la base de l'intervalle RAs,p-RVs (404) ; et
gérer la thérapie, fournie par le DMI (210), sur la base du retard AV qui est calculé.

2. DMI selon la revendication 1, dans lequel les un ou plusieurs processeurs (260) sont configurés pour gérer la thérapie en commandant l'électrode (222, 232) pour délivrer une thérapie de stimulation de la zone de la branche gauche du faisceau de His sur la base du retard AV.

3. DMI selon la revendication 1 ou 2, dans lequel le DMI (210) est un générateur d'impulsions implantable (GII) à double chambre.

4. DMI selon l'une quelconque des revendications 1 à 3, dans lequel les un ou plusieurs processeurs (260) sont configurés pour calculer le retard AV en soustrayant un pourcentage déterminé de l'intervalle RAs,p-RVs (404) à partir de l'intervalle RAs,p-RVs (404).

5. DMI selon l'une quelconque des revendications 1 à 4, dans lequel le signal d'AC (402) est un signal d'AC (402) intrinsèque.

6. DMI selon l'une quelconque des revendications 1 à 5, dans lequel les un ou plusieurs processeurs (260) sont en outre configurés pour :
après le calcul du retard AV, définir le retard AV comme un retard AV initial ; et
utiliser le retard AV initial pour gérer la thérapie pour un nombre déterminé de signaux d'AC.

7. DMI selon la revendication 6, dans lequel les un ou plusieurs processeurs (260) sont en outre configurés pour :
compter un nombre des signaux d'AC après un premier signal d'AC ;
comparer un nombre de signaux d'AC comptés au nombre déterminé de signaux d'AC ;
répéter la détermination de l'événement RAs,p (412), la détermination de l'événement RVs (414), la détermination de l'intervalle RAs,p-RVs (404) et le calcul du retard AV après que le nombre de signaux d'AC comptés correspond au nombre déterminé de signaux d'AC, pour calculer un retard AV mis à jour ; et
gérer la thérapie fournie par le DMI (210), sur la base du retard AV mis à jour.

8. DMI selon la revendication 7, dans lequel les un ou plusieurs processeurs (260) sont en outre configurés pour réinitialiser un compte du nombre de signaux d'AC comptés en réponse au calcul du retard AV mis à jour.

9. DMI selon l'une quelconque des revendications 1 à 7, dans lequel les un ou plusieurs processeurs (260) sont en outre configurés pour :
après le calcul du retard AV, déterminer un second intervalle RAs,p-RVs sur la base d'un second signal d'AC ;
comparer le second intervalle RAs,p-RVs au retard AV ; et
en réponse au fait que le second intervalle RAs,p-RVs est inférieur au retard AV, calculer un retard AV mis à jour sur la base du second intervalle RAs,p-RVs.

10. DMI selon la revendication 9, dans lequel les un ou plusieurs processeurs (260) sont en outre configurés pour :
compter un nombre de signaux d'AC obtenus après un premier signal d'AC,
comparer un nombre compté de signaux d'AC à un nombre déterminé de signaux d'AC ; et
réinitialiser le nombre compté des signaux d'AC en réponse au calcul du retard AV mis à jour.

11. DMI selon la revendication 9 ou 10, dans lequel les un ou plusieurs processeurs (260) sont en outre configurés pour gérer la thérapie, fournie par le DMI (210), sur la base du retard AV mis à jour, dans lequel les un ou plusieurs processeurs (260) sont configurés pour gérer la thérapie en commandant l'électrode (222, 224) pour délivrer une thérapie de stimulation de la zone de la branche gauche du faisceau de His sur la base du retard AV mis à jour.

12. DMI selon l'une quelconque des revendications 1 à 11, comprenant en outre une circuiterie de détection (230) configurée pour être couplée à l'électrode (222, 232) pour détecter le signal d'AC (402).

13. DMI selon l'une quelconque des revendications 1 à 12, comprenant en outre un générateur d'impulsions (270) commandé par les un ou plusieurs processeurs (260) pour délivrer des impulsions de stimulation de stimulation par l'intermédiaire de l'électrode (222, 232).

14. DMI selon la revendication 13, dans lequel les un ou plusieurs processeurs (260) sont en outre configurés pour commander le générateur d'impulsions (270) pour délivrer une impulsion de stimulation de stimulation par l'intermédiaire de l'électrode (222, 232) à l'expiration du retard AV.

15. DMI selon l'une quelconque des revendications 1 à 14, dans lequel les un ou plusieurs processeurs (260) sont configurés pour déterminer l'événement RVs (414) comme étant l'amplitude maximale (420) dans le signal d'AC (402) dans la période de temps déterminée (418) après que le signal d'AC (402) a atteint l'amplitude seuil (410).
